# EUROPEAN PATENT APPLICATION

(11) **EP 2 524 703 A1**
(43) Date of publication of application: **21.11.2012**
(21) Application number: 10842991.1
(22) Date of filing: 24.11.2010
(51) Int. Cl.: A61L 9/01, B01D 39/14, B01D 39/16, B01D 39/18, D04H 1/64, D06M 15/15

(54) **NON-WOVEN FABRIC FOR FILTER, AND PROCESS FOR PRODUCTION THEREOF**

(30) Priority: 15.01.2010 JP 2010007231
(71) Applicant: Kaneka Corporation, Osaka-shi, Osaka 530-8288 (JP)
(72) Inventor: YAMASHITA, Kenji, Settsu-shi Osaka 566-0072 (JP); YAGUCHI, Shigeru, Settsu-shi Osaka 566-0072 (JP); MURATA, Shoichi, Takasago-shi Hyogo 676-8688 (JP)
(74) Representative: Vossius & Partner
(86) International application number: PCT/JP2010/006849
(87) International publication number: WO 2011/086631

(57) **Abstract**

The present invention provides a multi-functional nonwoven fabric for filter that has excellent properties to adsorb various toxic/harmful substances such as odorous substances, VOCs, allergens, and pathogenic viruses, has antibacterial/antimold properties, and suppresses pressure loss to a minimal level, and the present invention also provides a method that can produce such a nonwoven fabric in a simple manner. Due to the simple steps of immersing the nonwoven fabric in an aqueous polypeptide solution and further treating it with a metal salt, it is possible to obtain a nonwoven fabric for filter having excellent adsorption properties in which the fiber surface of the nonwoven fabric is coated with insolubilized polypeptide that has been crosslinked with a metal. Also, a method that can produce such a filter in a simple manner was found.

## Description

### Technical Field

The present invention relates to a nonwoven fabric for filter that functions to simultaneously adsorb multiple harmful/toxic substances such as odorous substances, VOCs, allergens and viruses, has antibacterial/antimold properties, and also has the ability to suppress pressure loss to a minimal level, which is a fundamental property of a filter, and the present invention also relates to a production method therefor.

### Background Art

Applications of filters are for dust prevention, filtration, and air purification, and among such applications, the market of filters for air purification applications, for example, for air purifiers and air conditioners including car air conditioners has been expanding. Substances targeted by such filters mainly include odorous substances such as ammonia and isovaleric acid, toxic substances including formalin and like VOCs, various allergens such as tick-borne allergens that cause atopy and asthma, and various pathogenic viruses such as influenza virus and norovirus.

As described in Patent Document 1 in which a zeolite-based deodorizer is mixed with various resins such as acrylic resin and supported on the fiber surface, in Patent Document 2 in which a component that has virus inactivating action, such as catechins, is mixed with an aqueous resin and supported on a fiber, and in Patent Document 3 in which an enzyme that has allergen inactivating action is mixed with an aqueous resin and supported on a fiber, conventional filters intended for such multiple harmful/toxic substances are produced by selecting substances that are capable of adsorbing respective harmful/toxic substances, mixing them with a binder such as an aqueous synthetic resin, and then fixing them to a nonwoven fabric.

However, with such a method, in the case of production of a filter that removes multiple harmful/toxic substances, it is problematic in that multiple adsorbents need to be selected and employed, and the use of a binder causes adsorbents to be buried in the binder, thus not allowing sufficient adsorbability to be demonstrated, and moreover it is difficult to thinly coat the fiber surface when a conventional binder is used, resulting in a problem in that large pressure loss often accompanies.

### Citation List

### Patent Documents

Patent Document 1: JP 2007-260603A
Patent document 2: JP 2006-21095A
Patent document 3: JP 2003-210919A

### Disclosure of the Invention

### Problem to be Solved by the Invention

An object is to provide a nonwoven fabric that has excellent properties to adsorb various toxic/harmful substances such as odorous substances, VOCs, allergens, and pathogenic viruses, has antibacterial/antimold properties, and suppresses pressure loss to a minimal level, and also to provide a method that can produce such a nonwoven fabric in a simple manner.

### Means for Solving the Problem

The present invention is configured as follows.

1). A nonwoven fabric for filter obtained by treating a nonwoven fabric with an aqueous polypeptide solution having a polypeptide concentration of 0.01 to 8 wt% and further treating the fabric with a metal salt.

2). The nonwoven fabric for filter according to item 1, wherein metal of the metal salt is at least one selected from aluminum, zirconium, silver, copper, zinc, and titanium.

3). The nonwoven fabric for filter according to item 1 or 2, wherein a fiber surface of the nonwoven fabric is coated with insolubilized polypeptide that is crosslinked with the metal salt.

4). The nonwoven fabric for filter according to any of items 1 to 3, wherein the polypeptide is collagen.

5). The nonwoven fabric for filter according to any of items 1 to 4, wherein the nonwoven fabric is of a natural fiber, a chemical fiber, or a mixture of these.

6). A method for producing a nonwoven fabric for filter of any of items 1 to 5 having antibacterial/antimold properties, comprising treating a nonwoven fabric with an aqueous polypeptide solution having a polypeptide concentration of 0.01% to 8 wt% and further treating the nonwoven fabric with a metal salt.

### Effects of the Invention

The present invention provides a method that can produce in a simple manner a multi-functional nonwoven fabric for filter that has excellent properties to adsorb harmful/toxic substances such as odorous substances, VOCs, allergens, and pathogenic viruses, has antibacterial/antimold properties, and suppresses pressure loss to a minimal level.

### Brief Description of Drawings

[Fig. 1] An image of a nonwoven fabric observed under an electron microscope, which has been subjected to an immersion treatment in an aqueous collagen solution and then, without being pressed between rollers, subjected to a crosslinking treatment.
[Fig. 2] An image of a nonwoven fabric observed under an electron microscope, which has been treated with disrupted *E. coli.*
[Fig. 3] An image of a nonwoven fabric of the present invention observed under a phase-contrast microscope, which has been treated with mold spores.
[Fig. 4] An image of a conventional nonwoven fabric observed under a phase-contrast microscope, which has been treated with mold spores.

### Description of the Invention

In the following description, an example will be described in which solubilized collagen is for use as a polypeptide solution and an aluminum salt is for use as a metal salt used in crosslinking, but the present invention is not limited to these.

The present invention is a nonwoven fabric for filter having antibacterial/antimold properties obtained by treating a nonwoven fabric with an aqueous polypeptide solution having a polypeptide concentration of 0.01 to 8 wt% and further treating the fabric with a metal salt, and is also a production method therefor.

The fiber material formed into a nonwoven fabric in the present invention is not particularly limited as long as it can be formed into a fiber and processed into a nonwoven fabric form, and preferable examples include natural fibers, chemical fibers, and mineral fibers. Specific examples of natural fibers include cotton, sheep wool, hemp, pulp, silk, and the like.

Specific examples of chemical fibers include rayon, nylon, polyester, polypropylene, acrylic fibers, vinylon, aramid fibers, and the like. Specific examples of mineral fibers include glass fibers, carbon fibers, and the like. Among other things, natural fibers and/or chemical fibers are preferable in terms of ease of handling, cost, and the like. Specific examples include cotton, rayon, polyester, polypropylene, and pulp.

The "nonwoven fabric" preferably used in the present invention is a nonwoven fabric having an average fiber diameter of 50 nm to 100 µm. It is more preferable to provide a nonwoven fabric that uses fibers within this range, allows little pressure loss, has good gas permeability, and has as dense a structure as possible. Furthermore, in order for efficient adsorption of various harmful/toxic substances, it is desirable to make the fiber diameter as small as possible to increase the area of contact between various harmful/toxic substances and collagen that is insolubilized by crosslinking with a metal salt.

A fiber that has an extremely small fiber diameter is disadvantageous because the production thereof is difficult, thus leading to a cost increase, and pressure loss may increase depending on the fiber diameter and the mass per unit area adjustment. Therefore, a more preferable fiber diameter in the present invention is 500 nm to 50 µm, and even more preferably 1 µm to 20 µm.

The production method for a nonwoven fabric is roughly divided into a wet method that uses water and a dry method that does not use water, and more specifically, there are production processes as follows: namely, wet nonwoven fabric production, dry pulp nonwoven fabric production, dry nonwoven fabric production, spunbond nonwoven fabric production, melt-blown nonwoven fabric production, flashspun nonwoven fabric production, tow-opening nonwoven fabric production, and the like. In addition, there is also nonwoven fabric production by electrospinning. Note that the mass per unit area of the nonwoven fabric is preferably in the range of about 10 to 100 g/m² in terms of cost.

An aqueous polypeptide solution, when treated with a metal salt, is crosslinked by the metal salt, thus being insoluble in water. The metal salt is not limited as long as it can crosslink polypeptide. Specific examples of metals that can be used for metal salts include aluminum, zirconium, silver, copper, zinc, and titanium, and more specifically aluminum, zirconium, and copper, and in particular, aluminum and zirconium, and especially aluminum are preferable. It is preferable to use at least one of these metals.

In addition to collagen, gelatin, gelatin-decomposed polypeptide, modification products by a thermal or chemical treatment of collagen, gelatin, and gelatin-decomposed polypeptide, and like those that can be insolubilized by a metal salt or those that can retain a metal within the molecule and that can be water-soluble can be used as the polypeptide without any particularly limitation, and gelatin is preferable.

The insolubilized polypeptide in the present invention is, as described above, insolubilized matter insolubilized by crosslinking soluble polypeptide present in an aqueous polypeptide solution by a metal salt. The soluble polypeptide can be prepared using the skin of cows, pigs, horses, deer, rabbits, birds, fish, or like animals as well as the bone, tendon, scale, fish skin, or the like as an ingredient. Furthermore, it can be produced using, in particular, the split portion of the skin, bone, and tendon.

The split may be obtained from fresh split or salted rawhide obtainable from, for example cows, pigs, horses, deer, rabbits, birds, fish, or like animals. Such split is composed mostly of insoluble collagen fibers and is used after removing a fleshy portion that is adhered usually in a net-like manner and removing a salt that is used for preventing decomposition/deterioration.

Glycerides, phospholipids, free fatty acids, and like lipids, glycoproteins, albumins, and like proteins other than collagen, and similar impurities are present in insoluble collagen fibers. Such impurities greatly influence the quality such as luster and strength, odor, and the like when forming fibers. Therefore, it is preferable to remove such impurities in advance by hydrolyzing fat that is present in insoluble collagen fibers through liming to loosen collagen fibers and then performing a leather treatment that is generally carried out to cut the peptide portion, such as an acid/alkali treatment, an enzymatic treatment, a solvent treatment, or the like.

As the treatment method, a generally used known alkali treatment method, an enzymatic treatment method, a solvent treatment method, or the like is applicable. In the case where the alkali treatment method is applied, it is preferable to neutralize the alkali with an acid such as hydrochloric acid after the treatment. The method described in JP S46-15033B may be used as a known improved alkali treatment method.

Glycerides, phospholipids, free fatty acids, and like lipids, glycoproteins, albumins, and like proteins other than collagen, and similar impurities are present in insoluble collagen fibers. Such impurities greatly influence the quality such as luster and strength, odor, and the like when forming powders. Therefore, it is preferable to remove such impurities in advance by hydrolyzing fat that is present in insoluble collagen fibers through liming to loosen collagen fibers and then performing a leather treatment that is generally carried out, such as an acid/alkali treatment, an enzymatic treatment, a solvent treatment, or the like.

Treated insoluble collagen as stated above is subjected to a solubilization treatment to cut the crosslinked peptide portion. As a method for the solubilization treatment, a generally employed known alkali solubilization method, enzymatic solubilization method, or the like is applicable. In the case where the alkali solubilization method is applied, it is preferable to neutralize the alkali with an acid such as hydrochloric acid. The method described in JP S46-150338 may be used as a known improved alkali solubilization method.

The enzymatic treatment can yield collagen having a uniform molecular weight, and is thus a method that can be suitably employed in the present invention. As such an enzymatic treatment method, it is possible to employ methods that are described in, for example, JP S43-25829B and JP S43-27513B. Furthermore, the alkali treatment method and the enzymatic treatment method may be used in combination.

Carrying out such a treatment makes it possible to solubilize polypeptide. Further carrying out pH adjustment, salting out, water washing, solvent treatment, and like operations on the solubilized polypeptide can yield polypeptide and collagen of, for example, high quality, or that is, regarding collagen in particular, collagen that is largely composed of a triple-helix structure, which is the intrinsic structure of collagen can be obtained. The resulting polypeptide can form an aqueous solution, and in the present invention, the aqueous solution having a polypeptide concentration of 0.01 to 8 wt% is used. The aqueous solution can be used after being adjusted so as to be a solution having a concentration of preferably 0.03 to 3 wt%, particularly 0.04 to 0.4 wt%, and especially 0.04 to 0.3 wt%. It is preferable to use a solution having a concentration of 0.04 to 0.2 wt% in terms of imposing little influence on gas permeability. The aqueous polypeptide solution can be used as an aqueous polypeptide solution after dissolution using an acidic solution adjusted so as to have a pH of 2 to 4.5 with an acid such as hydrochloric acid, acetic acid, or lactic acid.

It is preferable to defoam the aqueous polypeptide solution by stirring it under reduced pressure. Also, it may be filtered to remove small water-insoluble unwanted particles. Moreover, suitable amounts of additives such as stabilizers and water-soluble polymeric compounds may be blended, as necessary with the aqueous polypeptide solution to, for example, enhance mechanical strength, enhance water resistance/heat resistance, improve luster, improve spinnability, prevent coloration, prevent degradation, and the like.

The solubilized polypeptide after being applied to the fiber surface is treated with a metal salt to crosslink the polypeptide, thus making the peptide water-insoluble and making it possible to fix the polypeptide to the fiber surface. It is preferable to use the metal salt in an aqueous solution form.

The treatment can be carried out by immersion in a metal salt-containing crosslinking fluid or spraying the crosslinking fluid or in a like manner. Immersion is preferable because it is possible to carry out the treatment uniformly. A padding method can also be used for immersion. As the metal salt, a salt with a strong acid such as sulfuric acid, a nitric acid salt, or a chloride can be used. Specifically, usable metals include aluminum, zirconium, silver, copper, zinc, and titanium. In particular, aluminum, zirconium and copper, and especially, aluminum are preferable. It is preferable to use at least one of these metals.

Examples of usable and preferable aluminum salts include basic aluminum chlorides and basic aluminum sulfates represented by Al(OH)ₙCl₃₋ₙ and Al₂(OH)₂ₙ(SO₄)₃₋ₙ, respectively, wherein n is 0.5 to 2.5.

Specifically, for example, aluminum sulfate, aluminum chloride, alum, and the like are used. These aluminum can be used singly or as a combination of two or more.

As for the metal salt concentration of the crosslinking fluid, 0.3 to 5 wt% in terms of metal oxide is preferable. Water is preferable as a solvent. When the metal oxide concentration is low, the metal salt content in polypeptide is high, thus making water resistance sufficient. When the metal oxide concentration is excessively high, the coated nonwoven fabric after the crosslinking treatment is highly likely to be hard and such a concentration is not preferable in terms of the handleability of the nonwoven fabric. The polypeptide-coated nonwoven fabric obtained by the present invention has properties to adsorb harmful/toxic substances.

Among the harmful/toxic substances, it is possible to favorably adsorb odorous substances, VOCs, allergens, pathogenic viruses, and bacteria/molds. The properties to adsorb harmful/toxic substances refer to the strength of affinity between the metal-crosslinked insolubilized polypeptide and harmful/toxic substances, i.e., a high rate of adsorption between harmful/toxic substances and the insolubilized polypeptide, the extent of adsorption capacity, and the like.

Odorous substances, VOCs, allergens, pathogenic viruses, and bacteria/molds targeted by the present invention will now be described below. Odorous substances include those contained in large amounts in food odor and feces odor, such as ammonia, trimethylamine, hydrogen sulfide, methyl mercaptan, and dimethyl disulfide, those regarded as sweat odor, such as acetic acid, isovaleric acid, acetaldehyde, and nonenal, etc.

VOCs are classified as follows. Those having a boiling point from 0°C or less to 50-100°C are very volatile organic compounds (VVOCs), those having a boiling point from 50-100°C to 240-260°C are volatile organic compounds (VOCs), those having a boiling point from 240-260°C to 400°C are semi volatile organic compounds (SVOCs), and those having a boiling point of 380°C or higher are particulate organic compounds (PVOCs), with VOCs being collectively referred to as semi volatile organic compounds (TVOCs). Among these, it is possible to favorably adsorb volatile organic compounds (VOCs). Specific volatile organic compounds include formaldehyde, toluene, xylene, p-dichlorobenzene, ethylbenzene, styrene, chlorpyrifos, di-2-ethylhexyl phthalate, diazinon, acetaldehyde, fenobucarb, and nonanal.

Generally, deodorizing or removal methods for odorous substances and VOCs can be roughly divided into three types, i.e., the adsorption type that uses an adsorbent such as activated carbon or zeolite; the catalyst type that decomposes and removes odorous substances with ozone, a photocatalyst, a metal-phthalocyanine complex, or the like; and the combination type that uses the adsorption type and the catalyst type in combination.

Substances that have physical adsorbing action and that can be used as adsorbents include activated carbon, activated earth, zeolite, sepiolite, silica gel, ceramics, activated alumina, composite phyllosilicates, and the like. Substances that have chemical adsorbing action include ion-exchange resins, iron-based compounds such as iron oxide, organic acids, and the like. Substances that have physical and chemical adsorbing action include impregnated charcoal, impregnated zeolite, natural inorganics, and the like. These conventional generally-used adsorbents can be used singly or as a mixture of multiple substances.

The function to remove odorous substances/VOCs of the nonwoven fabric of the present invention has the effect of removing the aforementioned odorous substances/VOCs when the nonwoven fabric is used as a filter, and this is primarily due to the adsorptive removal effect and/or decomposing effect of the insolubilized polypeptide itself, but it is also important to select the material of the nonwoven fabric depending on the target odorous substances/VOCs. Depending on the target odorous substances/VOCs, it is desirable to select a material that has stronger affinity with odorous substances/VOCs, such as chitosan fiber, which has a large amount of functional group such as an amino group.

Substances primarily serving as allergens are classified as follows. That is, there are, as ordinary allergens, house dust, which is an inhalant allergen (mainly dust mite's bodies, feces, and the like), skin debris (dandruff; in particular, dandruff of pets such as dogs and cats, and the like), pollen (cedar pollen, *Aln us firma* pollen, gramineous pollen, asteraceous pollen, and the like), fungi (mold and the like; in particular, *Alternaria),* insects (chironomids, cockroaches, and the like), stinging allergens (for example, bee sting), alimentary allergens (soybean, egg, cow milk, and the like), drug-based allergens (injected/orally administered; penicillin and the like), and the like, and as occupational allergens (through inhalation or contact), biological components/feces of animals, plant-based fine materials (flour, dust created during wood processing, and the like), and drugs (penicillin and the like).

Among these, typical allergens are pollen, which causes pollinosis; house dust, which causes perennial allergic rhinitis, bronchial asthma and atopic dermatitis; and the like. Fungi are also critical in bronchial asthma. It is well known that buckwheat in food allergy, bees (poison thereof), and the like are likely to lead to serious conditions, readily bringing about an anaphylactic shock.

The function to remove allergens of the nonwoven fabric for filter of the present invention has the effect of removing the aforementioned allergens, and this is primarily due to the adsorptive removal effect and/or decomposing effect of the insolubilized polypeptide itself, but it is possible to demonstrate a more significant effect by selecting a material that has affinity with the target allergen.

Pathogenic viruses refer to infectious viruses, and examples are as follows: human immunodeficiency virus (HIV), papillomavirus, molluscum contagiosum virus, wart virus, herpesvirus, influenza virus, parainfluenza virus, adenovirus, rhinovirus, coronavirus, Norwalk virus, rotavirus, echovirus, enterovirus, norovirus, carp herpesvirus, iridovirus, a rhabdo virus group, whitespot virus, and the like.

The antiviral function of the nonwoven fabric for filter of the present invention has the effect of inhibiting the aforementioned viruses, and this is primarily due to the adsorbing effect and/or decomposing effect of the insolubilized polypeptide itself, but it is possible to demonstrate a more significant effect by selecting a material that has affinity with the target virus.

Examples of bacteria and molds can be as follows. Meanwhile, germs are classified into bacteria and fungi, and usually there are few materials that are effective against both of these types, and a material that has such a function is desired. Generally, for classification of bacteria, bacteria are roughly divided as described below into Gram-positive bacteria, which have a large amount of peptidoglycan in the cell wall, Gram-negative bacteria, which have lipopolysaccharide, and other bacteria.

Gram-positive bacteria are further roughly divided into Gram-positive cocci and Gram-positive bacilli. Gram positive cocci include facultative anaerobic and aerobic cocci, and there are the genera *Micrococcus, Staphylococcus, Streptococcus,* and *Enterococcus Staphylococcus aureus* and methicillin-resistant *Staphylococcus aureus* (MRSA) of the genus *Staphylococcus,* and *Streptococcuspyogenes,* Group B Streptococcus, *Streptococcus pneumoniae,* and *Streptococcus viridans* of the genus *Streptococcus* are are known to be pathogenic bacteria.

Gram-positive bacilli are classified into the genera *Corynebacterium, Listeria, Erysipelothrix, Bacillus,* and *Mycobacterium.* Primary pathogenic bacteria include *Corynebacterium diphtheriae* of the genus *Corynebacterium; Listeria monocytogenes* of the genus *Listeria; Erysipelothrix rhusiopathiae* of the genus *Erysipelothrix; Bacillus anthracis* and *Bacillus cereus* of the genus *Bacillus;* and *Mycobacterium tuberculosis* of the genus *Mycobacterium.*

### Primary Gram-negative bacteria are Gram-negative bacilli.

Gram-negative bacilli include aerobic Gram-negative bacilli and Gram-negative facultative anaerobic bacilli.

Primary genera of aerobic Gram-negative bacilli include *Pseudomonas, Burkholderia, Rastonia, Legionella, Brucella, Bordetella, Alcaligenes, Francisella,* and the like. *Pseudomonas aeruginosa* of the genus *Pseudomonas; Legionella pneumophila* of the genus *Legionella; Brucella melitensis, Brucella abortus,* and *Brucella suis* of the genus *Brucella;* and the like are known to have pathogenicity.

Gram-negative facultative anaerobic bacilli are classified into the families *Enterobactenaceae, Vibrionaceae,* and *Pasteurellaceae,* and the family *Enterobacteriaceae* is further classified into the genera *Escherichia, Klebsiella, Serratia, Proteus,* and *Yersinia. Escherichia coli such* as O-157, salmonella, and dysentery bacillus of the genus *Escherichia; Klebsiella pneumoniae* of the genus *Klebsiella; Serratia marcescens* of the genus *Serratia, Proteus vulgaris* and *Proteus mirabilis* of the genus *Proteus;* and *Yersinia pestis* of the genus *Yersinia* are known to have pathogenicity. *Vibrio cholerae* of the genus *Vibrio* of the family *Vibrionaceae* and *Pasturella multocida* of the genus *Pasteurella* of the family *Pasteurellaceae* are known to be pathogenic bacteria.

Other bacteria include a group of bacteria that exist in the Gram-positive and negative forms, such as obligate anaerobes and spirilla, and known bacteria are as follows.

Obligate anaerobes are classified into obligate spore-forming bacteria, obligate anaerobic Gram-positive asporogenic bacilli, obligate anaerobic Gram-negative asporogenic bacilli, anaerobic Gram-positive cocci, and anaerobic Gram-negative cocci. Pathogenic bacteria include *Clostridium tetani, Clostridium botulinum, Clostridium perfringens,* and *Clostridium difficile,* which are obligate spore-forming bacteria.

Know pathogenic bacteria of a *Spirillaceae* group are *C. fetus, C. jijuni,* and *C. colit of* the genus *Campylobacter.*

The aforementioned various bacteria are known as pathogenic bacteria, and in particular, it can be said that *Escherichia coli, Staphylococcus aureus, Pseudomonas aeruglnosa,* MRSA, *Bacillus cereus,* and *Klebsiella pneumoniae,* which are often detected in food poisoning and hospital infection are highly significant as target bacteria of bactericidal agents.

### Next, fungi are roughly divided into yeasts and molds.

Molds are classified into the genus *Aspergillus,* the genus *Penicillum,* the genus *Cladosporium,* the genus *Alternaria,* the genus *Fusarium,* the genus *Aureobasidium,* the genus *Trichoderma,* and the genus *Chaetomium.* Targeted molds may be those that are indicated in JIS Z 2911, such as (first group) *Aspergillus niger, Aspergillus terreus,* and *Eurotium tonophilum;* (second group) *Penicillium citrinum* and *Penicillium funiculosum;* (third group) *Rhizopus oryzae;* (fourth group) *Cladosporium cladosporioides, Aureobasidium pullulans,* and *Gliocladium virens;* (fifth group) *Chaetomium globosum, Fusarium moniliforme,* and *Myrothecium verrucaria;* and the like.

Yeasts are classified into the genus *Canclida,* the genus *Rhodotorula,* and the genus *Saccharomyces* Other examples of fungi include dermatophytes and the like.

The antibacterial/antimold function of the nonwoven fabric for filter of the present invention has a growth inhibiting effect on the aforementioned bacteria and molds.

The production method for the above-described nonwoven fabric for filter, in which a nonwoven fabric is treated with an aqueous polypeptide solution and further treated with a crosslinking fluid containing a metal salt, first treats a nonwoven fabric with an aqueous polypeptide solution. The treatment method can be carried out by immersing a nonwoven fabric in an aqueous polypeptide solution or spraying an aqueous polypeptide solution onto a nonwoven fabric.

Immersing a nonwoven fabric in an aqueous polypeptide solution is preferable because it is possible to uniformly carry out the treatment. The fiber surface is clothed with an aqueous polypeptide solution and then treated with a metal salt solution, and as described above, the treatment method is carried out by, for example, immersion or spraying. In particular, the treatment by immersion is preferable because the treatment can be uniformly carried out.

The polypeptide clothed through this treatment, which is present in the aqueous polypeptide solution, is insolubilized, making it possible to fix the polypeptide to the fiber. Further, the nonwoven fabric is washed with water and dried to enable production. While the immersion method usually refers to immersing a nonwoven fabric in an aqueous polypeptide solution, it is possible to use a so-called padding method in which a nonwoven fabric is fed using rollers and continuously immersed in an aqueous polypeptide solution.

The polypeptide concentration of the aqueous polypeptide solution for treatment of a nonwoven fabric is 0.01 to 8 wt%, preferably 0.03 to 3 wt%, more preferably 0.03 to 0.4 wt%, and even more preferably 0.04 to 0.3 wt%. In particular, it is preferable to use a solution having a concentration of 0.04 to 0.2 wt%. When the concentration is within these ranges, the nonwoven fabric is preferable in that it does not result in impaired gas permeability (pressure loss) and can demonstrate adsorbability and biological activity as a filter. Temperature control is also important in the treatment of a nonwoven fabric with an aqueous polypeptide solution. The treatment is basically carried out at room temperature, but if room temperature significantly fluctuates, or if the polypeptide concentration is high or low, the treatment may also be carried out while adjusting the temperature so as to be in the range of 0 to 37°C.

The immersion time of the nonwoven fabric in an aqueous polypeptide solution is preferably such that the aqueous solution coats the entire fiber and then shifts to a crosslinking stage. Usually, it is desirable that immersion is carried out for 1 second to 6 hours, and more preferably for 1 minute to 1.5 hours.

Furthermore, shaking or applying mechanical force during immersion readily removes air and also enables efficient clothe of the fiber. As a means of more efficient clothe, a technique may be employed in which a clothe step is carried out during a step in which a nonwoven fabric and water simultaneously introduced into a highly-concentrated gelled aqueous collagen solution at room temperature and shaking is carried out to disperse and dissolve the gel over time.

Also, while it is sufficient that the nonwoven fabric removed from the aqueous solution at a stage where the process advances to a crosslinking treatment after immersion in the aqueous polypeptide solution is immersed as-is in a metal salt solution, it is also possible that the nonwoven fabric removed from the aqueous solution is subjected to shaking, centrifugal water removal, wringing, or a like treatment to remove the excessive aqueous solution, and then is immersed in a crosslinking fluid.

As for the procedure at the crosslinking stage, while the nonwoven fabric is basically left to stand still after being immersed in a crosslinking fluid, it is also possible to achieve clothed with insolubilized polypeptide by a method in which shaking is performed during the entire immersion time or part of the immersion time, a padding method in the nonwoven fabric after immersion is wrung with a pair of rolls, a method in which a crosslinking fluid is sprayed, or a combination of these methods.

At the crosslinking stage of the nonwoven fabric that has been immersed in an aqueous polypeptide solution, the metal salt solution is preferably acidic. Specifically, it is preferable to adjust the pH to 2.5 to 5. This pH adjustment can be carried out using, for example, hydrochloric acid, sulfuric acid, acetic acid, sodium hydroxide, sodium carbonate, or the like. With the metal salt solution being in such a state, the polypeptide structure can be favorably retained, the metal salt does not precipitate, and the metal salt solution readily permeates uniformly.

It is preferable that the pH is initially adjusted to a slightly low pH so as to allow the aqueous metal salt solution to sufficiently permeate throughout regenerated collagen, and then the pH is adjusted to a slightly high pH by adding, for example, sodium hydroxide, sodium carbonate, or the like to complete the treatment. Specifically, it is possible that the pH is initially adjusted to 2.2 to 3.5 and then to 3.5 to 5 to complete the treatment.

In the case where a highly basic metal salt such as polyaluminum chloride is used, only the initial pH adjustment to 2.5 to 5 may be carried out. The fluid temperature of the metal salt solution is not particularly limited, and it is preferably 50°C or lower. When the fluid temperature is 50°C or lower, denaturation or deterioration of polypeptide is unlikely to occur.

The time of immersing in the metal salt solution the nonwoven fabric that has been treated with an aqueous polypeptide solution is preferably 1 second or longer, and more preferably 1 minute to 25 hours. If the immersion time is excessively short, the reaction with the metal salt may be insufficient, possibly resulting in a problematic water resistance of polypeptide. There is no particular limitation on the upper limit of the immersion time, and with the reaction time of about 1 hour, the reaction with the metal salt sufficiently proceeds, bringing about favorable water resistance. In order to prevent a nonuniform concentration resulting from rapid absorption of the metal salt into polypeptide, an inorganic salt such as sodium chloride, sodium sulfate, or potassium chloride may be added to the aqueous metal salt solution.

Insolubilized polypeptide fixed to the fiber surface of the present invention also functions as a binder, and it is possible during collagen immersion or at a crosslinking stage after collagen immersion to mix with a conventional offensive odor and VOC removing substance, allergen removing substance, or antiviral substance described in the previous section, thus making it possible to fix such substances to the fiber surface, and it is thus possible to further enhance the offensive odor/VOC removing properties, allergen removing properties, antibacterial/antimold properties, or antiviral properties of the nonwoven fabric for a functional filter of the present invention.

A preferable metal that can be used for a metal salt is preferably at least one metal selected from the aforementioned metals. It is an effective means in the present invention that in the case where, for example, aluminum or zirconium, or in particular aluminum, is selected, a metal salt of these metals and a metal salt of at least one metal selected from silver, copper, and zinc are mixed or used in combination. In particular, use of silver in combination is preferable because antibacterial, antimold, allergen removing, and antiviral properties become favorable.

The higher the polypeptide concentration, the more likely the aforementioned properties become favorable, while the gas permeability of the filter is lowered. However, when a solution having a polypeptide concentration of 0.04 to 0.3 wt% is used, crosslinking of collagen using aluminum and at least one metal selected from silver, copper, and zinc in combination results in a better balance between polypeptide concentration and gas permeability than singly using aluminum or zirconium, or in particular aluminum, and is thus preferable. In particular, use of a solution having a collagen concentration of 0.04 to 0.2 wt% brings about the same permeability as that of an unprocessed nonwoven fabric that has not received any crosslinking treatment with polypeptide, while favorable antibacterial, antimold, allergen removing, and antiviral properties are retained, and is thus preferable.

As for the ratio of aluminum or zirconium, or in particular aluminum, to at least one metal selected from three metals, i.e., silver, copper, and zinc, the number of atoms of the at least one metal selected from three metals, i.e., silver, copper, and zinc per atom of aluminum or zirconium, or in particular aluminum, is preferably from 1/30 to 1/3000, and more preferably from 1/100 to 1/1000.

The nonwoven fabric obtained by present invention is used for a filter. Applications of the filter is to separate gas and solid, separate liquid and solid, and allow fluid to permeate and volatilize. The filter can be used for vehicles as a car air-conditioner filter, an air filter, and an oil filter for air-conditioning of buildings or the like; for clean rooms where an ultrahigh performance is required; and for house-hold products such as air conditioners and air purifiers.

Also, the nonwoven fabric can be used for garment applications as lab equipment/protective clothing; environmental protection applications as a water cleaning filter; an article of daily use or hygiene product such as a mask and a sanitary item; fabric-based furniture/vehicle seat; interior materials such as fabric-based wallpaper; electric appliances; printing applications; and paper products. In addition, it can be used for the field of cosmetics, food products, and pharmaceuticals.

When the nonwoven fabric of the present invention is used for a filter, it is possible to suppress pressure loss, and thus the nonwoven fabric can be suitably used for a filter. The term "pressure loss" refers to the pressure difference between the inlet-side pressure and the outlet-side pressure of a filter. Measurement is usually carried out by a method in accordance with JIS B 9908.

The insolubilized polypeptide of the present invention can be used such that it is processed into a suitable form such as fine powder and then mixed with various binder resins, or is added to a nonwoven fabric after a binder is adhered to the nonwoven fabric in advance. Note that any resin can be used as a binder resin.

Examples include self crosslinked acrylic resin, methacrylic resin, urethane resin, silicone resin, glyoxal resin, vinyl acetate resin, vinylidene chloride resin, butadiene resin, melamine resin, epoxy resin, acryl-silicon copolymer resin, ethylene-vinyl acetate copolymer resin, isobutylene-maleic anhydride copolymer resin, ethylene-styrene-(meth)acrylate copolymer resin, and the like. The binder resin may be a mixture of two or more of these resins.

### Examples

Hereinbelow, the present invention shall be described in detail by way of examples, but the present invention is not limited thereto. Note that, measurement methods of the characteristic values presented in examples will be described below. Also, in the examples below, the term "ordinary temperature" refers to "15 to 25°C" and the symbol "%" refers to "wt%" unless specified otherwise. The polypeptide concentration was calculated by a procedure in which the amount of nitrogen was measured by the Kjeldahl method and then converted into the amount of protein.

### Production Example

### (Production Example 1) Production of nonwoven fabric clothed with aluminum-crosslinked insolubilized collagen

30 g of an aqueous hydrogen peroxide solution that had been diluted to 30 wt% was introduced into 1200 kg of pieces of cow hide derived from cow split as a raw material (corresponding to 180 kg of collagen) and solubilized with an alkali, and the cow hide was dissolved in an aqueous lactic acid solution, thus giving a stock adjusted so as to have a pH of 3.5 and a solid content of 7.5%. The stock was subjected to an agitational defoaming treatment by an agitational defoamer (manufactured by Dalton Co., Ltd., 8DMV) under reduced pressure, transferred to a piston spinning stock tank, and left to stand still under reduced pressure to defoam, thus giving a collagen solution for the following nonwoven fabric treatment.

A 5 cm × 12 cm synthetic fiber nonwoven fabric AL035J11-GN-H (manufactured by Kinsei Seishi Co., Ltd., a weight of 35 g/m²) was immersed in 300 ml of an aqueous collagen solution adjusted so as to have a concentration of 0.024%, 0.06%, 0.12%, or 0.18% by diluting the above-described collagen solution for nonwoven fabric with water, shaken at room temperature for 1 hour, and then immersed without modification in an aluminum crosslinking fluid (a pH of 4) composed of 80 g of trisodium citrate dihydrate, 84.8 g of sodium hydroxide, 960 g of an aqueous aluminum sulfate (a concentration of 8% in terms of Al₂O₃) solution, 1278 g of sodium sulfate, and 7536 g of water. The nonwoven fabric was left to stand still at room temperature overnight, washed with a sufficient amount of water, then air-dried.

Next, the extent of collagen coating over the nonwoven fabric was calculated based on the weight increase of the nonwoven fabric caused by immersion in the aqueous collagen solution and the aluminum crosslinking treatment. In comparison, aluminum-crosslinked nonwoven fabric on which no immersion treatment was carried out in an aqueous collagen solution was prepared, and the weight increase thereof was measured. Results are shown in Table 1.

### (Production Example 2) Production of nonwoven fabric clothed with aluminum-crosslinked insolubilized collagen

A nonwoven fabric clothed with insolubilized collagen was obtained in the same manner as in Production Example 1 except that a pulp-blended fiber nonwoven fabricAL040 TCEP-WE (manufactured by Kinsei Seishi Co., Ltd. a weight of 40 g/m²) was immersed in an aqueous collagen solution adjusted so as to have a collagen concentration of 0.06% or 0.12% as in Production Example 1.

Next, the extent of collagen coating over the nonwoven fabric was calculated based on the weight increase of the nonwoven fabric caused by immersion in the aqueous collagen solution and the aluminum crosslinking treatment. In comparison, aluminum-crosslinked nonwoven fabric on which no immersion treatment was carried out in an aqueous collagen solution was prepared, and the weight increase thereof was measured. Results are shown in Table 1.

### (Production Example 3) Production of nonwoven fabric clothed with aluminum-crosslinked insolubilized collagen

A nonwoven fabric clothed with insolubilized collagen was obtained in the same manner as in Production Example 1 except that a synthetic fiber nonwoven fabric 21GP (manufactured by Kinsei Seishi Co., Ltd. a weight of 20 g/m²) was immersed in an aqueous collagen solution adjusted so as to have a collagen concentration of 0.06% or 0.12% as in Production Example 1.

Next, the collagen coverage over the nonwoven fabric was calculated based on the weight increase of the nonwoven fabric caused by immersion in the aqueous collagen solution and the aluminum crosslinking treatment. In comparison, aluminum-crosshnked nonwoven fabric on which no immersion treatment was carried out in an aqueous collagen solution was prepared, and the weight increase thereof was measured. Results are shown in Table 1.

### (Production Example 4) Production of nonwoven fabric clothed with aluminum-crosslinked insolubilized collagen

A nonwoven fabric clothed with insolubilized collagen was obtained in the same manner as in Production Example 1 except that a rayon fiber nonwoven fabric 3020 (manufactured by Kinsei Seishi Co., Ltd. a weight of 20 g/m²) was immersed in an aqueous collagen solution adjusted so as to have a collagen concentration of 0.06% or 0.12% as prepared in Production Example 1.

Next, the insolubilized collagen coverage over the nonwoven fabric was calculated based on the weight increase of the nonwoven fabric caused by immersion in the aqueous collagen solution and the aluminum crosslinking treatment. In comparison, aluminum-crosslinked nonwoven fabric on which no immersion treatment was carried out in an aqueous collagen solution was prepared, and the weight increase thereof was measured. Results are shown in Table 1.

### (Production Example 5) Production of nonwoven fabric clothed with thermally-treated insolubilized collagen

A non-woven fabric was obtained according to Production Example 1 except that the aqueous solution used was an aqueous collagen solution having a collagen concentration of 0.18% as produced in Production Example 1 that had been left to stand at 60°C for 30 minutes to facilitate gelatinization of collagen.

Next, the insolubilized collagen coverage over the nonwoven fabric was calculated based on the weight increase of the nonwoven fabric caused by immersion in the aqueous collagen solution and the aluminum crosslinking treatment. In comparison, aluminum-crosslinked nonwoven fabric on which no immersion treatment was carried out in an aqueous collagen solution was prepared, and the weight increase thereof was measured. Results are shown in Table 1.

[Table 1]

**(Table 1)**

| | | | | | | |
|---|---|---|---|---|---|---|
| Concentration (%) of treatment aqueous collagen solution | | 0 | 0.024 | 0.06 | 0.12 | 0.18 |
| Production Example 1 | Weight increase (%) caused by collagen/Al treatment | 0 | 0.72 | 1.36 | 5.1 | 6.51 |
| Production Example 2 | | 0 | - | 1.21 | 4.83 | - |
| Production Example 3 | | 0 | - | 1.14 | 4.54 | - |
| Production Example 4 | | 0 | - | 1.25 | 4.94 | - |
| Production Example 5 | | 0 | - | - | - | 1.65 |

### (Production Example 6)

The collagen solution for nonwoven fabric treatment prepared in Production Example 1 was diluted with water to give an aqueous polypeptide solution having a collagen concentration of 0.25%. Five pieces of a synthetic fiber nonwoven fabric AL035J11-GN-H (manufactured by Kinsei Seishi Co., Ltd., a weight of 35 g/m²) having a length of 1 m per side were immersed in the foregoing aqueous solution for about 10 seconds, pressed with nip rolls at a feeding rate of 5 m/min at a nip pressure of 0.2 MPa, and then immersed in an aluminum crosslinking fluid (a pH of 4) composed of 80 g of trisodium citrate dihydrate, 84.8 g of sodium hydroxide, 960 g of an aqueous aluminum sulfate (a concentration of 8% in terms of Al₂O₃) solution, 1278 g of sodium sulfate, and 7536 g of water for 10 seconds to carry out an insolubilization treatment. Thereafter, the nonwoven fabric was washed with a sufficient amount of water and dried at 60°C to give a polypeptide-coated nonwoven fabric. The coating amount (the increased weight) was 170 mg/m².

### (Production Example 7)

Apolypeptide-coated nonwoven fabric was obtained by carrying out a treatment, washing with a sufficient amount of water, and air-drying in the same manner as in Production Example 6 except that an aqueous polypeptide solution adjusted so as to have a collagen concentration of 0.5% by diluting the collagen solution for nonwoven fabric treatment prepared in Production Example 1 with water was used and the immersion time in an aluminum crosslinking fluid (a pH of 4) was 10 minutes. The coating amount was 410 mg/m².

### (Production example 8)

A polypeptide-coated nonwoven fabric was obtained in the same manner as in Production Example 6 except that an aqueous polypeptide solution adjusted so as to have a collagen concentration of 2.0% by diluting the collagen solution for nonwoven fabric treatment prepared in Production Example 1 with water was used. The coating amount was 720 mg/m².

### (Production Example 9)

A polypeptide-coated nonwoven fabric was obtained in the same manner as in Production Example 6 except that an aqueous polypeptide solution adjusted so as to have a collagen concentration of 0.25% by diluting the collagen solution for nonwoven fabric treatment prepared in Production Example 1 with water was used and an aluminum crosslinking fluid (a pH of 4) admixed with silver nitrate so as to have a concentration of 0.5 mM was used. The coating amount was 520 mg/m².

### (Production Example 10)

A polypeptide-coated nonwoven fabric was obtained in the same manner as in Production Example 9 except that an aqueous polypeptide solution adjusted so as to have a gelatin (reagent: manufactured by Wako Pure Chemical Industries, Ltd.) concentration of 0.3% was used and the immersion time in an aluminum crosslinking fluid (a pH of 4) was one night. The coating amount was 100 mg/m².

### (Production Example 11)

A polypeptide-coated nonwoven fabric was obtained in the same manner as in Production Example 10 except that an aqueous polypeptide solution adjusted so as to have a collagen concentration of 0.3% by diluting the collagen solution for nonwoven fabric treatment prepared in Production Example 1 with water was used and an aqueous solution composed of 300 g of sodium sulfate, 55.6 g of zirconium sulfate (manufactured by Daiichi Kigenso Kagaku Kogyo Co., Ltd.), and 1644 g of water was used as a crosslinking fluid. The coating amount was 1000 mg/m².

### (Production Example 12)

Apolypeptide-coated nonwoven fabric was obtained in the same manner as in Production Example 10 except that an aqueous polypeptide solution adjusted so as to have a collagen concentration of 0.3% by diluting the collagen solution for nonwoven fabric treatment prepared in Production Example 1 with water was used and an aqueous solution composed of 300 g of sodium sulfate, 12.5 g of copper (II) sulfate pentahydrate, and 1687.5 g of water was used as a crosslinking fluid. The coating amount was 750 mg/m².

Accordingly, the extent of insolubilized collagen or gelatin coating over the nonwoven fabrics was calculated based on the weight increase of the nonwoven fabric caused by immersion in the aqueous collagen solution and the crosslinking treatment with aluminum, zirconium, copper, or aluminum and silver. In comparison, aluminum-crosslinked nonwoven fabric on which no immersion treatment was carried out in an aqueous collagen solution was prepared, and the weight increase thereof was measured.

### Production Examples 13 to 15

Aqueous polypeptide solutions at 15°C having a collagen concentration of 0.05% (Production Example 13), 0.1% (Production Example 14), and 0.15% (Production Example 15) were prepared by diluting the collagen solution for nonwoven fabric treatment prepared in Production Example 1 with water. A synthetic fiber nonwoven fabric AL035J11-GN-H (manufactured by Kinsei Seishi Co., Ltd., a weight of 35 g/m²) that had been cut so as to have a width of 40 cm and a length of 80 cm was immersed in these aqueous solutions for 5 minutes, pressed with nip rolls at a feeding rate of 5 m/min at a nip pressure of 0.2 MPa, and then immersed in an aluminum crosslinking fluid (a pH of 4) composed of 80 g of trisodium citrate dihydrate, 84.8 g of sodium hydroxide, 960 g of an aqueous aluminum sulfate (a concentration of 8% in terms of Al₂O₃) solution, 1278 g of sodium sulfate, and 7536 g of water overnight to carry out an insolubilization treatment. Thereafter, the nonwoven fabric was washed with a sufficient amount of water and dried at 60°C to give a polypeptide-coated nonwoven fabric. Properties of the resulting nonwoven fabric are shown in Table 9.

### Production Example 16

### Production of nonwoven fabric in which silver was also introduced in aluminum-crosslinked insolubilized collagen

A polypeptide-coated nonwoven fabric was obtained in the same manner as in Production Example 15 except that an aqueous polypeptide solution adjusted so as to have a collagen concentration of 0.15% by diluting the collagen solution for nonwoven fabric treatment prepared in Production Example 1 with water was used and an aluminum crosslinking fluid (a pH of 4) admixed with silver nitrate so as to have a concentration of 0.5 mM was used. The coating amount was 220 mg/m² (Table 9).

### Comparative Production Example 1

A 9% collagen solution was prepared according to Production Example 1 while reducing the amount of the aqueous lactic acid solution used. A polypeptide-coated nonwoven fabric was obtained in the same manner as in Production Example 13 except that a nonwoven fabric was immersed in the aqueous solution for 10 seconds. A large amount of collagen film was observed between fibers in the resulting nonwoven fabric, and the nonwoven fabric lacked drapability and had an appearance that is highly unsuitable for use as a filter.

### Example 1

### Analysis of coating on fiber

An analysis of coating was carried out using an SEM-EDS apparatus for electron microscope observation of the fiber surface of the nonwoven fabric produced in Production Example 1, the extent of coating with insolubilized collagen, and the like. With those treated at a collagen concentration exceeding 0.18%, a large amount of film-like matter was observed between nonwoven fabric fibers within one field of view in electron microscope observation. With those treated at a concentration of 0.12% or less, no film-like matter was observed.

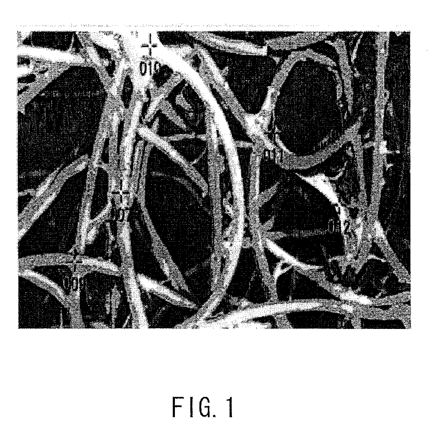

Those treated at a concentration of 0.12% and, as a control, a nonwoven fabric subjected to only an aluminum crosslinking treatment without immersion in an aqueous collagen solution were analyzed. Here, the extent of coating was calculated from the viewpoint of the amount of aluminum element on the fiber surface. Fig. 1 is an electron microscope image of a nonwoven fabric subjected to an immersion treatment in an aqueous collagen solution, and the reference numerals therein indicate where coating was analyzed. That is, the amount of aluminum was measured with an SEM-EDS apparatus at five places, i.e., 007, 009, 010, 011, and 012.

As a result, no difference was observed between the fiber diameter of the nonwoven fabric of Production Example 1, which was subjected to an immersion treatment in a 0.12% aqueous collagen solution, and the fiber diameter of the nonwoven fabric not treated with collagen, but the aluminum analysis by SEM-EDS detected aluminum over the entire fiber of the nonwoven fabric of Production Example 1, showing that the fiber was entirely coated with insolubilized collagen. On the other hand, no aluminum was detected from the control. Hence, as a result of a fiber coating treatment by the present method, a nonwoven fabric that had a substantially unchanged appearance such as the fiber diameter of the nonwoven fabric and that had a fiber surface coated with insolubilized collagen was obtained. Since the appearance, e.g., fiber diameter, of the nonwoven fabric is unchanged, the pressure loss of the filter is suppressed to a minimal level.

The following tests evaluated the extent of properties to remove various harmful/toxic substances (odorous substances, VOCs, allergens, pathogenic viruses, and the like) gained by the nonwoven fabric whose fiber surface was coated with insolubilized polypeptide by the present method.

### Example 2

### Test of properties to remove odors/VOCs

### (Properties to remove ammonia odor)

Ammonia gas was injected so as to reach a concentration of 30 ppm into a 5 L TEDLAR bag in which the nonwoven fabric sample (20 cm × 10 cm) prepared in Production Example 1 by an immersion treatment in a 0.12% aqueous collagen solution was placed, and the residual concentration of ammonia gas was measured over time using a detector tube. The total amount of ammonia gas removed was calculated from the measured value, and accordingly the rate (%) of ammonia gas removal was calculated. Results are shown in Table 2.

### (Properties to remove acetic acid odor)

The rate (%) of acetic acid gas removal was calculated in the same manner as in the measurement of properties to remove ammonia odor described above except that acetic acid gas was introduced in place of ammonia gas into a TEDLAR bag so as to reach a concentration of 10 ppm. Results are shown in Table 2.

### (Properties to remove isovaleric acid odor)

The rate (%) of acetic acid gas removal was calculated in the same manner as in the measurement of properties to remove ammonia odor described above except that isovaleric acid gas was introduced in place of ammonia gas into a TEDLAR bag so as to reach a concentration of 15 ppm. Results are shown in Table 2.

### (Properties to eliminate acetaldehyde odor)

The rate (%) of acetaldehyde removal was calculated in the same manner as in the measurement of properties to remove ammonia odor described above except that acetaldehyde gas was introduced in place of ammonia gas into a TEDLAR bag so as to reach a concentration of 20 ppm. Results are shown in Table 2.

### (Properties to eliminate formaldehyde odor)

The rate (%) of acetaldehyde removal was calculated in the same manner as in the measurement of properties to remove ammonia odor described above except that formaldehyde gas was introduced in place of ammonia gas into a TEDLAR bag so as to reach a concentration of 20 ppm. Results are shown in Table 2.

Note that, in the odor/VOC removal tests described above, an untreated nonwoven fabric not treated with polypeptide was used for comparison in each removal test.

[Table 2]

**(Table 2)**

| Target | Sample | Sampling time (hr) | | | |
|---|---|---|---|---|---|
| | | 0 | 1 | 3 | 24 |
| Ammonia | Collagen/Al-treated nonwoven fabric | 0 | 97 | 100 | - |
| | Untreated nonwoven fabric | 0 | 30 | 33 | - |
| Acetic acid | Collagen/Al-treated nonwoven fabric | 0 | 93 | 100 | - |
| | Untreated nonwoven fabric | 0 | 72 | 85 | - |
| Isovaleric acid | Collagen/Al-treated nonwoven fabric | 0 | 77 | 83 | 100 |
| | Untreated nonwoven fabric | 0 | 73 | 77 | 88 |
| Acetaldehyde | Collagen/Al-treated nonwoven fabric | 0 | 15 | 23 | 25 |
| | Untreated nonwoven fabric | 0 | 10 | 15 | 18 |
| Hormaldehyde | Collagen/Al-treated nonwoven fabric | 0 | 60 | 70 | 80 |
| | Untreated nonwoven fabric | 0 | 15 | 30 | 40 |

### Example 3

### Test of properties to remove allergens

### (Test using mite allergen)

Properties to remove an allergen of the nonwoven fabric produced in Production Example 1 by an immersion treatment in a 0.12% aqueous collagen solution was determined using a mite allergen assay kit Derf1 ELISA Kit (manufactured by Funakoshi Corporation). That is, properties to remove a mite-derived allergen (*Dermatophagoides farinae,* manufactured by Funakoshi Corporation) by adsorption were evaluated by the ELISA method using an anti-mite allergen antibody.

The aforementioned nonwoven fabric, having a size of 2 cm × 6 cm, was immersed in 750 µl of a mite allergen antigen solution adjusted to 20 ng/ml, and left to stand at room temperature for 1 hour. Thereafter, the non-woven fabric was removed, and the amount of mite allergen in the remaining solution was measured by the ELISA method to calculate the rate of mite allergen adsorption of the nonwoven fabric. For comparison, properties to remove the allergen of an untreated nonwoven fabric were measured. Results are shown in Table 3.

### (Test using disrupted E. coli)

Properties to remove an allergen of the nonwoven fabric produced in Production Example 1 by an immersion treatment in a 0.12% aqueous collagen solution were evaluated from the view point of the extent of adsorption on the nonwoven fabric of disrupted *E. coli* that was generated as a result of viral infection of *E. coli.* That is, the Qβ virus (coliphage Qβ NBRC20012) infection of *E*. *coli* (NBRC13965) disrupted this *E. coli,* and as a result, disrupted matter in a fine particle form was generated.

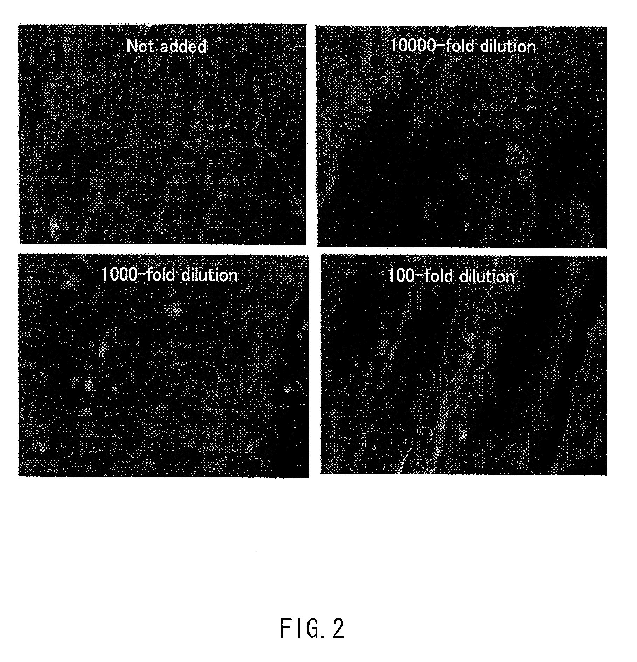

The aforementioned nonwoven fabric was immersed in a solution in which the disrupted matter was diluted in a stepwise manner, left to stand still at room temperature for 1 hour, and washed. Thereafter, the nonwoven fabric fiber was observed under an electron microscope to determine adsorption. Results are shown in Fig. 2. It was observed that, in an inversely correlated manner, the higher the degree of dilution of the disrupted matter, the fewer the fine particles adhered to the surface.

### (Test of mold spore removal)

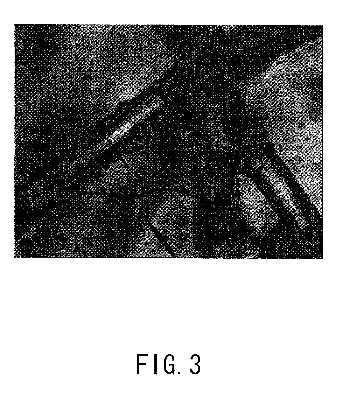

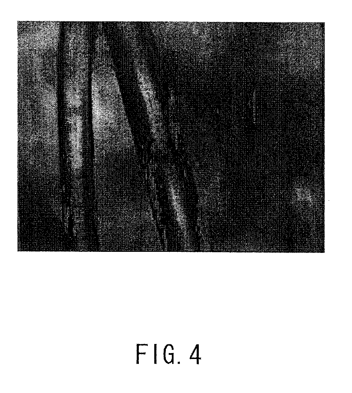

Properties to remove an allergen of the nonwoven fabric produced in Production Example 1 by an immersion treatment in a 0.12% aqueous collagen solution were evaluated from the view point of adsorption of mold spores (the genus *Penicillium)* on the nonwoven fabric. That is, the nonwoven fabric having a size of 1 cm per side was immersed for 1 hour in 1 ml of a solution adjusted so as to have 3 × 10⁶ mold spores/ml, and washed with water. Thereafter, adhesion of mold spores to the fiber surface of the nonwoven fabric was observed under a phase-contrast microscope (Fig. 3). For comparison, an observation of the fiber surface of an untreated nonwoven fabric that had been treated in the same manner was carried out (Fig. 4). As a result, it was observed that a significant amount of spores were adhered to the collagen-treated nonwoven fabric, while adhesion was barely observed on the untreated nonwoven fabric.

### (Test of mold spore injury)

Properties to remove an allergen of the nonwoven fabric produced in Production Example 1 by an immersion treatment in a 0.12% aqueous collagen solution were evaluated from the view point of the extent of growth of mold spores (the genus *Penicillium)* adhered to the nonwoven fabric. That is, the nonwoven fabric having a size of 1 cm per side was immersed for 1 hour in 1 ml of a solution adjusted so as to have 1 × 10⁶ mold spores/ml, and washed with water. Thereafter, the nonwoven fabric was placed on nutrient agar, and growth of the mold in an environment where the nonwoven fabric was left to stand still at room temperature was evaluated. The evaluation of growth was carried out by measuring the length to the tip of a hypha extending from the edge of the nonwoven fabric.

For comparison, the same operation was carried out on a nonwoven fabric that had been immersed in an aqueous collagen solution and air-dried, and growth of a hypha was measured. A result of leaving the nonwoven fabrics to stand still for 3 days, significant hypha growth was observed on the nonwoven fabric treated only in the aqueous collagen solution, while growth was barely observed on the nonwoven fabric that had been subjected to immersion in the aqueous collagen solution as well as an aluminum crosslinking treatment.

### Example 4

### Test of antiviral properties

### (Test using Qβ virus)

The antiviral properties of the nonwoven fabric produced in Production Example 1 by an immersion treatment in a 0.12% aqueous collagen solution was determined using a Qβ virus (coliphage Qβ NBRC20012), which is an *E. coli* virus. The nonwoven fabric having a size of 4 cm × 0.5 cm was immersed in 10 ml of a virus suspension adjusted so as to have a virus titer of 2.4 × 10⁸ plaque forming unit (pfu)/ml, and left to stand still at room temperature for 1 hour. Thereafter, the viral titer in the immersion fluid was determined by a plaque formation method using E. *coli* (NBRC 13965). For comparison, antiviral properties of an untreated nonwoven fabric that had not been treated with collagen were evaluated in the same manner. Results are shown in Table 3.

### (Test using feline calicivirus)

Furthermore, antiviral properties of the nonwoven fabric produced in Production Example 1 (immersed in a 0.12% aqueous collagen solution and crosslinked by aluminum) were determined from the view point of the adsorptive removability of feline calicivirus (feline calicivirus vaccine strain), which is regarded as a norovirus model. That is, the nonwoven fabric having a size of 2 cm × 2 cm was immersed in 250 µl of a viral solution adjusted so as to have an infectivity titer of 10^{5.7} (a viral load necessary to infect 50% of 1 ml of cells), and left to stand still at room temperature for 1 hour. Thereafter, the nonwoven fabric was removed, and the infectivity titer of the remaining solution was measured to calculate the rate of virus adsorption by the nonwoven fabric.

As for the viral infectivity titer, culture cell CRFK cells were monolayer-cultured in a tissue culture microplate (96 wells), then a cell growth medium (Eagle MEM with 10% fetal bovine serum) was removed, and a cell maintenance medium (Eagle MEM with 2% fetal bovine serum) was added in an amount of 0.1 ml each.

Next, 0.1 ml of a test fluid was inoculated in each of 4 wells and cultured for 4 to 7 days in a 37°C carbon dioxide gas incubator (CO₂: 5%). After culturing, the presence or absence of a morphological change of the cells was determined with an inverted phase-contrast microscope, and the median tissue culture infectious dose was calculated by the Reed-Muench method and converted into the viral infectivity per milliliter of the test fluid. Results are shown in Table 3.

[Table 3]

**(Table 3)**

| | Removed substance | Sample | Rate (%) of removal |
|---|---|---|---|
| Ex. 3 | Mite allergen | Collagen/Al-treated nonwoven fabric | 95 |
| | | Untreated nonwoven fabric | 30 |
| Ex. 4 | Qβ virus | Collagen/Al-treated nonwoven fabric | 98 |
| | | Untreated nonwoven fabric | 32 |
| | Feline calicivirus | Collagen/Al-treated nonwoven fabric | 97 |
| | | Untreated nonwoven fabric | 27 |

### Example 5

### (Test of antibacterial properties using E. coli)

The nonwoven fabrics produced in Production Examples 1 to 4 by an immersion treatment in a 0.12% aqueous collagen solution were cut into small pieces so as to have a size that allowed 100 mg of insolubilized collagen to be contained, and the antibacterial properties of the nonwoven fabrics were evaluated. That is, the pieces of the nonwoven fabrics were each immersed in a 10 ml L-broth medium (0.5% yeast extract, 1% peptone, 0.5% common salt). Thereafter, *E. coli* (IFO 3972) was inoculated and cultured at 37°C overnight. After culturing, the presence or absence of proliferation was visually determined, For comparison, an untreated nonwoven fabric that had not been treated with collagen was cultured under the same conditions. Results are shown in Table 4.

### (Test of antimold properties using Cladospotium cladosporioides)

The nonwoven fabric produced in Production Example 1 by an immersion treatment in a 0.12% aqueous collagen solution was cut into small pieces so as to have sizes that allowed 125 mg and 62.5 mg of collagen to be contained, and the antimold properties of the nonwoven fabric were evaluated. That is, 10 ml of water was added to the pieces of the nonwoven fabric, *Cladosporium cladosporioides* (IFO 6348) was inoculated, and a shaking treatment was carried out at 25°C for one day. After the treatment, supernatant was spread onto the same SABOURAUD AGAR (manufactured by Eiken Chemical Co., Ltd.) and cultured at 25°C for seven days. The presence or absence of proliferation was determined with the naked eye. An untreated nonwoven fabric that had not been treated with collagen was cultured under the same conditions. Results are shown in Table 4. In the determination of the presence or absence of mold proliferation, in the case where the culture medium appeared turbid, it was determined that proliferation was present.

[Table 4]

**(Table 4)**

| Proliferation test | Material of nonwoven fabric | Treatment | Presence or absence of proliferation |
|---|---|---|---|
| *E. coli* | AL035J11-GN-H Prod. Ex. 1 | Collagen/Al-treated nonwoven fabric | Absent |
| | | Untreated nonwoven fabric | Present |
| | AL040TCEP-WE Prod. Ex. 2 | Callagen/Al-treated nonwoven fabric | Absent |
| | | Untreated nonwoven fabric | Present |
| | 21GP Prod. Ex. 3 | Collagen/Al-treated nonwoven fabric | Absent |
| | | Untreated nonwoven fabric | Present |
| | 21GP Prod. Ex. 4 | Collagen/Al-treated nonwoven fabric | Absent |
| | | Untreated nonwoven fabric | Present |
| *Cladosporium cladospoioides* | AL035J11-GN-H | Collagen/Al-treated nonwoven fabric (12.5 mg/ml) | Absent |
| | | Collagen/Al-treated nonwoven fabric (6.25 mg/ml) | Absent |
| | | Untreated nonwoven fabric | Present |

### Example 6

### Test of allergen removing properties

### (Test using mite allergen Derf2)

Properties to remove an allergen of the nonwoven fabric produced in Production Example 1 by an immersion treatment in a 0.12% aqueous collagen solution was determined using a mite-derived allergen antigen Derf2 (manufactured by Asahi Breweries Ltd). Properties to remove a mite allergen by adsorption were evaluated by the ELISA method using an anti-mite allergen antibody. The collagen-treated nonwoven fabric having a size of 2 cm × 6 cm was immersed in 750 µl of a mite allergen antigen solution adjusted to 500 ng/ml, and left to stand at room temperature for 24 hour. Thereafter, the nonwoven fabric was removed, and the amount of mite allergen in the remaining solution was measured by the ELISA method to calculate the rate of mite allergen removal of the nonwoven fabric. Results are shown in Table 5.

[Table 5]

**(Table 5) Evaluation of properties to remove mite allergen (Derf2)**

| Collagen treatment on nonwoven fabric | Derf2 (ng/ml) | Rate (%) of removal |
|---|---|---|
| Not performed | 500 | 0 |
| Performed | 0.6 | 99.9 |

### (Test using cedar pollen allergen Cryj1)

Properties to remove an allergen of the nonwoven fabric produced in Production Example 1 by an immersion treatment in a 0.12% aqueous collagen solution was determined using a cedar pollen allergen antigen Cryj1 (manufactured by Seikagaku Corporation). Properties to remove a cedar pollen allergen were evaluated by the ELISA method using an anti-cedar pollen allergen antibody.

The collagen-treated nonwoven fabric having a size of 2 cm × 6 cm was immersed in 750 µl of a celar pollen allergen solution adjusted to 50 ng/ml, and left to stand at room temperature for 30 minutes or 24 hours. Thereafter, the nonwoven fabric was removed, and the amount of mite allergen in the remaining solution was measured by the ELISA method to calculate the rate of cedar pollen allergen removal of the nonwoven fabric. Results are shown in Table 6.

[Table 6]

**(Table 6) Evaluation of properties to remove cedar pollen allergen (Cryj1)**

| Collagen treatment on nonwoven fabric | Cryj1 (ng/ml) | | Rate (%) of removal | |
|---|---|---|---|---|
| Not performed | 50 | | 0 | |
| Performed | 0.5 hours | 24 hours | 0.5 hours | 24 hours |
| | 1.3 | 0.43 | 97.4 | 99.1 |

### Example 7

### Test of antiviral properties

### (Test using influenza A virus)

Antiviral properties of the nonwoven fabric produced in Production Example 1 (immersed in a 0.12% aqueous collagen solution and crosslinked by aluminum) were evaluated from the view point of the rate of influenza A (H1N1) inactivation. That is, the nonwoven fabric having a size of 2 cm × 2 cm was immersed in 250 µl of a viral solution adjusted so as to have an infectivity titer of 10^{6.5} (a viral load necessary to infect 50% of 1 ml of cells), and left to stand still at room temperature for 24 hours. Thereafter, the solution was recovered, and the infectivity titer of the recovered solution was measured to calculate the rate of virus inactivation by the nonwoven fabric treatment.

A culture cell MDCK (NBL-2) cell ATCC CCL-3 strain was monolayer-cultured in a tissue culture flask, then a cell growth medium (Eagle MEM with 10% fetal bovine serum) was removed, and a test virus was inoculated. Next, a cell maintenance medium (Eagle MEM with 2% fetal bovine serum) was added, and cultured for 1 to 5 days in a 37°C carbon dioxide gas incubator (CO₂: 5%). After culturing, the presence or absence of a morphological change of the cells was determined with an inverted phase-contrast microscope, and the median tissue culture infectious dose was calculated by the Reed-Muench method and converted into the viral infectivity per milliliter of the test fluid.

The test operation was carried out in the following manner. A sample cut so as to have a size of about 2 cm × 2 cm was placed in a petri dish, and 0.25 ml of a virus suspension was added dropwise. Thereafter, the sample was transferred to another petri dish and left to stand still at room temperature. After the petri dish was left to stand still for 24 hours, the suspension that had been added dropwise was recovered, and conversion to viral infectivity was carried out in the same manner as described above. Results are shown in Table 7.

[Table 7]

**(Table 7) Test of influenza A inactivation**

| Test virus | Treatment of nonwoven fabric | LogTCID₅₀/ml |
|---|---|---|
| Influenza A | Performed | 4.5 |
| | Not performed | 6.5 |

| | | |
|---|---|---|
| TCID50: Median tissue culture infectious dose. The degree of dilution to attain the amount of virus necessary to infect 50% of cultured tissue | | |

### Example 8

### Test of antibacterial properties

Antibacterial properties of the nonwoven fabrics produced in Production Examples 6 to 12 were evaluated by a procedure in accordance with JIS L 1902. The target bacterium used was *E. coli (IFO* 3972). For comparison, an untreated nonwoven fabric that had not been treated with polypeptide was processed and evaluated under the same conditions. Results are shown in Table 8.

### Example 9

### Test of antimold properties

Antimold properties of the nonwoven fabrics produced in Production Examples 6 to 9 were evaluated by a procedure in accordance with JIS Z 2911 (mold resistance test). Four mold species were used, i.e., *Aspergillus niger, Penicillium citrinum, Chaetomium globosum,* and *Myrothecium verrucaria.* For comparison, an untreated nonwoven fabric that had not been treated with polypeptide was processed and evaluated under the same conditions. Results are shown in Table 8.

### Example 10

### Test of antiviral properties

### (Test using Qβ virus)

Antiviral properties of the nonwoven fabrics produced in Production Examples 6 to 9 were evaluated using a Qβ virus (coliphage Qβ NBRC20012), which is an *E. coli* virus. The nonwoven fabrics each having a size of 2 cm × 2 cm was inoculated with 125 ml of a virus suspension adjusted so as to have a virus titer of 3 × 10⁶ plaque forming unit (pfu)/ml, and left to stand still at room temperature for 1 hour. Thereafter, the viral titer in the immersion fluid was determined by a plaque formation method using E. coli (NBRC 13965). For comparison, antiviral properties of an untreated nonwoven fabric that had not been treated with polypeptide were evaluated in the same manner. Results are shown in Table 8.

### Example 11

### Test of properties to remove allergen

### (Test using mite allergen Derf2)

Properties to remove an allergen of the nonwoven fabrics produced in Production Example 6 to 9 were determined using a mite-derived allergen antigen Derf2 (manufactured by Asahi Breweries Ltd). Properties to remove a mite allergen were evaluated by the ELISA method using an anti-mite allergen antibody. The four corners of each nonwoven fabric (5 cm × 5 cm) were secured with clips and set so as to be suspended in air.

0.5 ml of a mite allergen antigen solution adjusted so as to reach 500 ng/ml was allowed to be uniformly absorbed throughout the nonwoven fabric. Thereafter, the apparatus was placed in a plastic container, saturated brine was poured into the bottom of the plastic container, and the lid was placed. The plastic container was left to stand still at room temperature for 1 hour while maintaining humidity. After the plastic container was left to stand for 1 hour, 0.5 ml of distilled water was allowed to be absorbed into the nonwoven fabric, the fluid was extracted by squeezing the nonwoven fabric by hand, and the Derf2 concentration in the extract was measured by the ELISA method. For comparison, an untreated nonwoven fabric that had not been treated with polypeptide was processed in the same manner, and its properties to remove the allergen were evaluated. Results are shown in Table 8.

[Table 8]

**(Table 8)**

| | Antivirus property test | Antimold property test | Allergen removal property test | Antivirus property test |
|---|---|---|---|---|
| Nonwoven fabric | Viable cell count (cfu/ml)* | Growth** | Rate of allergen removal | Virus infectivity titer (pfu/ml)*** |
| (Prod. Ex. 6) | 5.4 × 10⁴ | Yes (+++) | > 99.6% | 3.6 × 10³ |
| (Prod. Ex. 7) | < 3 × 10³ | Yes (++) | > 99.6% | 4.7 × 10³ |
| (Prod. Ex. 8) | < 3 × 10³ | Yes (+) | > 99.6% | 7.2 × 10² |
| (Prod. Ex. 9) | < 3 × 10³ | No | > 99.6% | 2.2 × 10³ |
| (Prod. Ex. 10) | < 3 × 10³ | - | - | - |
| (Prod. Ex. 11) | 8.5 × 10³ | - | - | - |
| (Prod. Ex. 12) | 3.8 × 10⁵ | - | - | - |
| Untreated nonwoven fabric | 5.8 × 10⁷ | Yes (++++) | 91% | 9.2 × 10⁵ |

| | | | | |
|---|---|---|---|---|
| *: The viable cell count was converted into the viable cell concentration of a bacterial suspension inoculated with a sample. cfu: colony forming unit **: ++++ extreme growth, +++ significant growth, ++ growth, + slight growth ***: The virus infectivity titer was converted into the virus concentration of a virus suspension inoculated with a sample. pfu: plaque forming unit | | | | |

### Example 12

### Test of antibacterial properties

Antibacterial properties of the nonwoven fabrics produced in Production Examples 6 to 9 and 13 to 16 were evaluated by a procedure in accordance with JIS L 1902. The target bacterium used was *E. coli* (IFO 3972). For comparison, an untreated nonwoven fabric that had not been treated with polypeptide was processed and evaluated under the same conditions. Results are shown in Table 9.

### Example 13

### Test of antimold properties

Antimold properties of the nonwoven fabrics produced in Production Examples 6 to 9 and 13 to 16 were evaluated by a procedure in accordance with JIS Z 2911 (a mold resistance test). Four mold species were used, i.e., *Aspergillus niger, Penicillium citrinum, Chaetomium globosum,* and *Myrothecium verrucaria.* For comparison, an untreated nonwoven fabric that had not been treated with polypeptide was processed and evaluated under the same conditions. Results are shown in Table 9.

### Example 14

### Test of antiviral properties

Antiviral properties of the nonwoven fabrics produced in Production Examples 6 to 9 and 13 to 16 were evaluated using a Qβ virus (coliphage Qβ NBRC20012), which is an *E. coli* virus. The nonwoven fabrics each having a size of 2 cm × 2 cm was inoculated with 0.125 ml of a virus suspension adjusted so as to have a virus titer of 3 × 10⁶ plaque forming unit (pfu)/ml, and left to stand still at room temperature for 1 hour. Thereafter, the viral titer in the immersion fluid was determined by a plaque formation method using *E. coli* (NBRC 13965). For comparison, antiviral properties of an untreated nonwoven fabric that had not been treated with polypeptide were evaluated in the same manner. Results are shown in Table 9.

### Example 15

### Test of properties to remove allergens

Properties to remove an allergen of the nonwoven fabrics produced in Production Example 6 to 9 and 13 to 16 were determined using a mite-derived allergen antigen Derf2 (manufactured by Asahi Breweries Ltd). Properties to remove a mite allergen were evaluated by the ELISA method using an anti-mite allergen antibody. The four corners of each nonwoven fabric (5 cm × 5 cm) were secured with clips and set so as to be suspended in air. 0.5 ml of a mite allergen antigen solution adjusted so as to reach 500 ng/ml was allowed to be uniformly absorbed throughout the nonwoven fabric. Thereafter, the apparatus was placed in a plastic container, saturated brine was poured into the bottom of the plastic container, and the lid was placed. The plastic container was left to stand still at room temperature for 1 hour while maintaining humidity. After the plastic container was left to stand for 1 hour, 0.5 ml of distilled water allowed to be absorbed into the nonwoven fabric, the fluid was extracted by squeezing the nonwoven fabric by hand, and the Derf2 concentration in the extract was measured by the ELISA method. For comparison, an untreated nonwoven fabric that had not been treated with polypeptide was processed in the same manner, and its properties to remove the allergen were evaluated. Results are shown in Table 9.

### Example 16

### Gas permeability test

The gas permeability of the nonwoven fabrics produced in Production Example 6 to 9 and 13 to 16 was measured using a gas permeability tester KES-F8-AP1 (manufactured by Kato Tech Co., Ltd.). For measurement, 8 places on each processed nonwoven fabric (8 cm × 15 cm) were randomly selected and subjected to measurement, with the tester being set to its measurement range of H, and their average value was presented. Results are shown in Table 9.

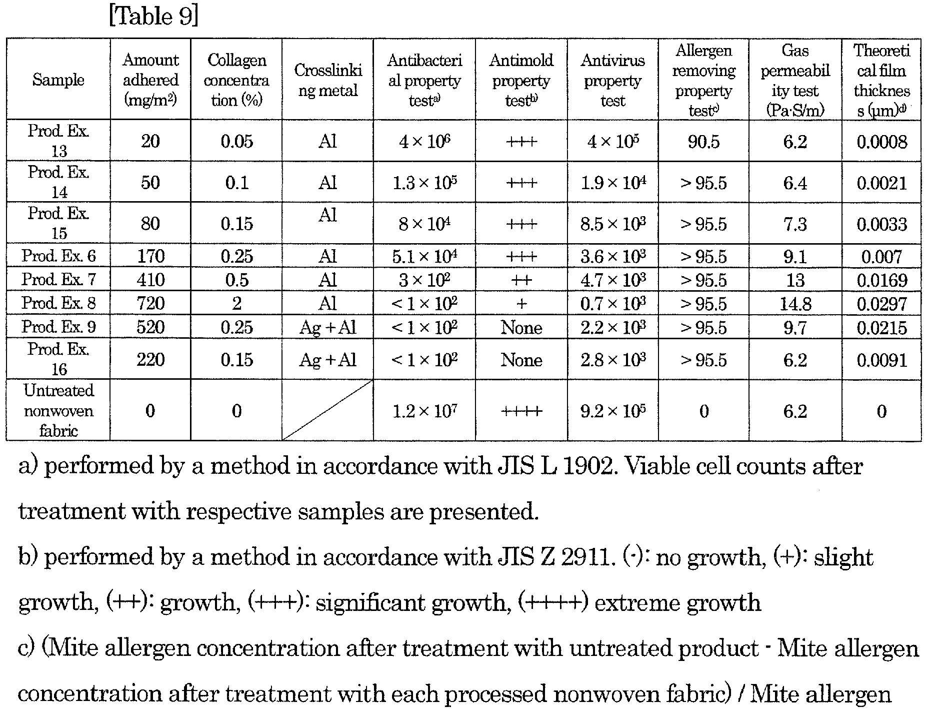

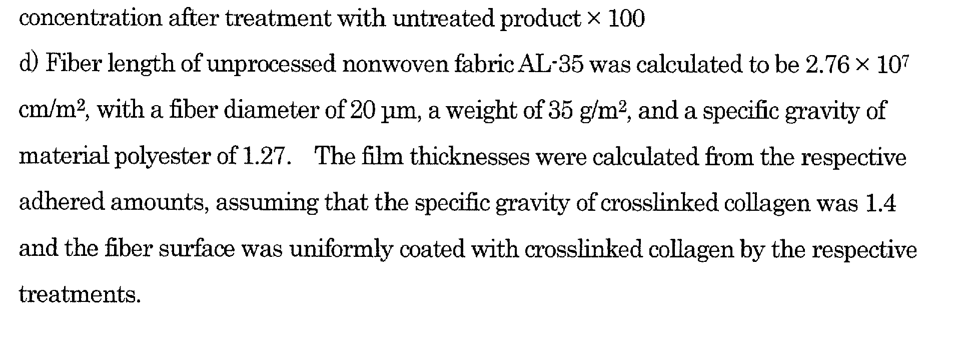

## Claims

1. A nonwoven fabric for filter obtained by treating a nonwoven fabric with an aqueous polypeptide solution having a polypeptide concentration of 0.01 to 8 wt% and further treating the fabric with a metal salt.

2. The nonwoven fabric for filter according to claim 1, wherein metal of the metal salt is at least one selected from aluminum, zirconium, silver, copper, zinc, and titanium.

3. The nonwoven fabric for filter according to claim 1 or 2, wherein a fiber surface of the nonwoven fabric is coated with insolubilized polypeptide that is crosslinked with the metal salt.

4. The nonwoven fabric for filter according to any of claims 1 to 3, wherein the polypeptide is collagen.

5. The nonwoven fabric for filter according to any of claims 1 to 4, wherein the nonwoven fabric is of a natural fiber, a chemical fiber, or a mixture of these.

6. A method for producing a nonwoven fabric for filter of any of claims 1 to 5 having antibacterial/antimold properties, comprising treating a nonwoven fabric with an aqueous polypeptide solution having a polypeptide concentration of 0.01% to 8 wt% and further treating the nonwoven fabric with a metal salt.
